(19) Europäisches Patentamt
European Patent Office
Office européen des brevets

(11) EP 3 822 261 A1

(12) EUROPEAN PATENT APPLICATION
published in accordance with Art. 153(4) EPC

(43) Date of publication:
**19.05.2021 Bulletin 2021/20**

(21) Application number: **19834810.4**

(22) Date of filing: **04.06.2019**

(51) Int Cl.:
***C07D 307/20*** *(2006.01)* ***B01J 31/28*** *(2006.01)*
***C07B 61/00*** *(2006.01)*

(86) International application number:
**PCT/JP2019/022241**

(87) International publication number:
**WO 2020/012830 (16.01.2020 Gazette 2020/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **13.07.2018 JP 2018133166**

(71) Applicant: **Kuraray Co., Ltd.**
**Kurashiki-shi, Okayama 710-0801 (JP)**

(72) Inventors:
- **SHIMIZU,Ryosuke**
**Kamisu-shi, Ibaraki 314-0197 (JP)**
- **NAKAGAWA, Hiroyuki**
**Tainai-shi, Niigata 959-2691 (JP)**

(74) Representative: **D Young & Co LLP**
**120 Holborn**
**London EC1N 2DY (GB)**

# (54) CYCLIC HEMIACETAL COMPOUND PRODUCTION METHOD

(57) Provided is a method for producing a specific hemiacetal compound, the method including reacting a specific alcohol having an unsaturated bond, carbon monoxide, and hydrogen in the presence of a compound of Group 8 to Group 10 transition metal, a tertiary phosphite ligand, and a base, wherein an A value represented by the equation (1) is 0.0 to 4.0, and a B value represented by the equation (2) is 0.6 to 1.0.



Printed by Jouve, 75001 PARIS (FR)

**Description**

Technical Field

The present invention relates to a method for producing a cyclic hemiacetal compound.

Background Art

A method for producing an aldehyde by reacting an unsaturated compound with hydrogen and carbon monoxide is called a hydroformylation reaction (or oxo reaction) and is industrially useful. In the hydroformylation reaction, a cobalt compound or a rhodium compound is usually used as a catalyst.

It is known to produce a cyclic hemiacetal compound by hydroformylating an alcohol having an unsaturated bond, and a large number of examples have been reported. For example, a method is known in which allyl alcohol (PTL 1), methallyl alcohol (PTL 2), or 3-methyl-3-butene-1-ol (PTL 3) is hydroformylated to synthesize a corresponding cyclic hemiacetal compound.

Meanwhile, a transition metal catalyst used as a catalyst is generally expensive, and the cyclic hemiacetal compound and the catalyst component may be separated by an operation such as single evaporation to reuse the catalyst. For example, PTL 4 describes that when an aldehyde is produced by hydroformylating an olefinic compound, a specific bisphosphite compound having high heat resistance is used as a ligand, whereby the bisphosphite compound is stably present without decomposition or deterioration even in a separation step of a reaction product, and a reaction solution after separation of the reaction product can be recycled to a reactor for producing an aldehyde as it is.

Citation List

Patent Literature

PTL 1: JP 5-320087 A
PTL 2: JP 9-52888 A
PTL 3: JP 62-201881 A
PTL 4: JP 11-130720 A

Summary of Invention

Technical Problem

In the methods for producing cyclic hemiacetal compounds disclosed in PTLs 1 to 3, there is still room for improvement in the yield. In addition, the cyclic hemiacetal compound produced in the hydroformylation reaction often lacks stability, and many high-boiling components are likely to be contained in the resulting reaction mixture. However, when the reaction mixture contains such many high-boiling components, the recovery rate is deteriorated when the target cyclic hemiacetal compound is separated by single evaporation or the like from the reaction mixture. In particular, in the case where the catalyst is reused as described above, when the reaction mixture contains many high-boiling components, the content of the high-boiling components increases as the number of times of reuse increases, and the recovery rate further deteriorates, and thus the formation of such a high-boiling component may be a cause of hindering the reuse of the catalyst. Therefore, it is required to suppress high-boiling components in the reaction mixture.

In view of the above problems, it is an object of the present invention to provide a method for producing a cyclic hemiacetal compound in a simple manner and in a good yield under mild conditions while suppressing the formation of high-boiling components.

Solution to Problem

The present inventors have found that the above problems can be solved by adopting specific reaction conditions when a specific alcohol having an unsaturated bond is hydroformylated to produce a specific cyclic hemiacetal compound, and have completed the present invention through further studies based on the findings.

That is, the present disclosure relates to the following [1] to [7].

[1] A method for producing a hemiacetal compound represented by the following general formula (II) (hereinafter

sometimes referred to as "cyclic hemiacetal compound (II)"), the method including reacting an alcohol having an unsaturated bond represented by the following general formula (I) (hereinafter sometimes referred to as "unsaturated alcohol (I)"), carbon monoxide, and hydrogen in the presence of a compound of Group 8 to Group 10 transition metal, a tertiary phosphite ligand, and a base, wherein an A value represented by the following equation (1) is 0.0 to 4.0, and a B value represented by the following equation (2) is 0.6 to 1.0:

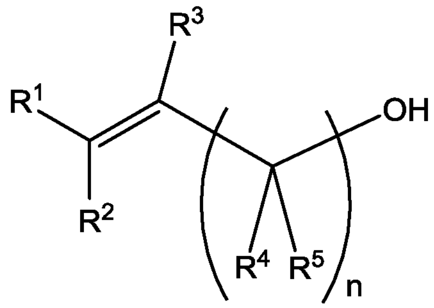

(I)

wherein $R^1$ to $R^5$ each independently represent any one selected from the group consisting of a hydrogen atom, an optionally substituted alkyl group having 1 to 6 carbon atoms, and an optionally substituted aryl group having 6 to 12 carbon atoms, and n represents 1 or 2;

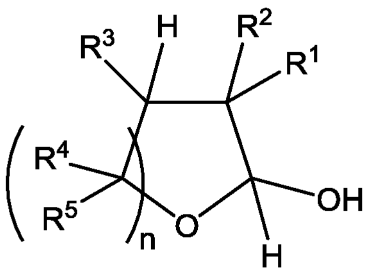

(II)

wherein $R^1$ to $R^5$ and n are as defined above;

$$A = \frac{120000 \times \exp\left(-\frac{13000}{T}\right) \times (1-0.16\ln x)}{120 \times \exp\left(-\frac{13000}{T}\right) \times (1-0.16\ln x) + 9.8 \times \exp\left(-\frac{10000}{T}\right) \times (1-0.075\ln x)} \quad (1)$$

$$B = 1 - \exp\left\{-4.9 \times 10^{12} \times \exp\left(-\frac{10000}{T}\right) \times [M] \times (1-0.075\ln x) \times t\right\} \quad (2)$$

wherein T represents a reaction temperature (K), x represents a concentration (mM) of a base in the reaction system (wherein x is 500 or less), [M] represents a concentration (mM) of a transition metal atom of Group 8 to Group 10 derived from a compound of Group 8 to Group 10 transition metal in the reaction system, and t represents a reaction time (hr) in a batch reaction or a residence time (hr) in a continuous reaction.

[2] The method according to [1], wherein the compound of Group 8 to Group 10 transition metal is a rhodium compound.

[3] The method according to [1] or [2], wherein $R^1$ and $R^2$ in the general formula (I) are a hydrogen atom.

[4] The method according to any one of [1] to [3], wherein the alcohol having an unsaturated bond represented by the general formula (I) is methallyl alcohol or 3-methyl-3-butene-1-ol.

[5] The method according to any one of [1] to [4], wherein the tertiary phosphite ligand is a triaryl phosphite.

[6] The method according to any one of [1] to [4], wherein the tertiary phosphite ligand is represented by the following general formula (III):

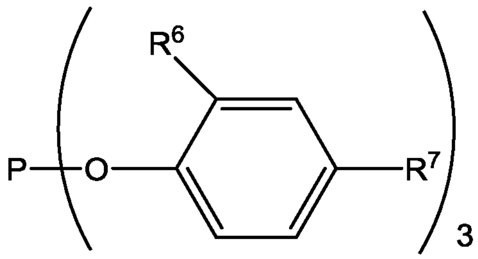

(III)

wherein $R^6$ and $R^7$ each independently represent any one selected from the group consisting of a hydrogen atom and an optionally substituted alkyl group having 1 to 6 carbon atoms.

[7] The method according to any one of [1] to [6], wherein the base is a tertiary amine.

Advantageous Effects of Invention

According to the present invention, there is provided a method capable of producing a cyclic hemiacetal compound in a simple manner and in a good yield under mild conditions while suppressing the formation of high-boiling components.

Description of Embodiments

In the method of the present invention for producing a cyclic hemiacetal compound (II), an unsaturated alcohol (I), carbon monoxide, and hydrogen are reacted in the presence of a compound of Group 8 to Group 10 transition metal, a tertiary phosphite ligand and a base (hereinafter, the reaction may be sometimes referred to as "hydroformylation reaction"). In the method of the present invention, the A value represented by the following equation (1) is 0.0 to 4.0, and the B value represented by the following equation (2) is 0.6 to 1.0.

$$A = \frac{120000 \times \exp\left(-\frac{13000}{T}\right) \times (1-0.16 \ln x)}{120 \times \exp\left(-\frac{13000}{T}\right) \times (1-0.16 \ln x) + 9.8 \times \exp\left(-\frac{10000}{T}\right) \times (1-0.075 \ln x)} \quad (1)$$

$$B = 1 - \exp\left\{-4.9 \times 10^{12} \times \exp\left(-\frac{10000}{T}\right) \times [M] \times (1-0.075 \ln x) \times t\right\} \quad (2)$$

In the equation (1) and the equation (2), T represents a reaction temperature (K), x represents a concentration (mM) of a base in the reaction system (wherein x is 500 or less), [M] represents a concentration (mM) of a transition metal atom of Group 8 to Group 10 derived from a compound of Group 8 to Group 10 transition metal in the reaction system, and t represents a reaction time (hr) in a batch reaction or a residence time (hr) in a continuous reaction.

[A Value and B Value]

The A value represented by the above equation (1) relates to the selectivity of the cyclic hemiacetal compound (II), and the lower the A value is, the higher the selectivity of the cyclic hemiacetal compound (II) tends to be. In the present invention, the A value needs to be in the range of 0.0 to 4.0, and this can suppress the formation of a high-boiling component contained in the reaction mixture. When the A value exceeds 4.0, the content of the high-boiling component contained in the reaction mixture is increased, and there is a possibility that the reuse of the catalyst becomes difficult. From such a viewpoint, the A value is preferably 3.5 or less, more preferably 3.0 or less, still more preferably 2.5 or less, and particularly preferably 2.2 or less.

The A value is a function of the reaction temperature T (unit: K) and the concentration x (unit: mM) of the base in the reaction system. For example, when the reaction temperature T is increased, the A value is increased, and when the concentration x of the base is increased, the A value is decreased.

The B value represented by the above equation (2) relates to the conversion rate of the unsaturated alcohol (I), and the higher the B value is, the higher the conversion rate of the unsaturated alcohol (I) tends to be. In the present invention, it is necessary that the B value is 0.6 to 1.0, and thereby, the target cyclic hemiacetal compound (II) can be

obtained in a good yield while suppressing the formation of a high-boiling component contained in the reaction mixture. When the B value is less than 0.6, the conversion rate of the unsaturated alcohol (I) is decreased, and the yield of the target cyclic hemiacetal compound (II) is decreased. From such a viewpoint, the B value is preferably 0.70 or more, more preferably 0.75 or more, and still more preferably 0.80 or more.

The B value is a function of the reaction temperature T (unit: K), the concentration x (unit: mM) of the base in the reaction system, the concentration [M] (unit: mM) of the transition metal atom of Group 8 to Group 10 derived from the compound of Group 8 to Group 10 transition metal in the reaction system, and the reaction time or the residence time t (unit: hr). For example, when the reaction temperature T or the concentration [M] of the transition metal atom of Group 8 to Group 10 derived from the compound of Group 8 to Group 10 transition metal in the reaction system is increased, the B value is increased. Further, even if the reaction time or the residence time t is prolonged, the B value tends to be increased. On the other hand, when the concentration x of the base in the reaction system is increased, the B value tends to be decreased.

In the above equations (1) and (2), when the reaction temperature is not constant, the average value obtained by weighting each reaction temperature with time may be used as the reaction temperature T. For example, when the reaction is performed at the temperature T1 (K) for 2 hours and then at the temperature T2 (K) for 1 hour, the average value $(T1 \times 2 + T2 \times 1)/3$ may be used as the reaction temperature T. Similarly, when the concentration x of the base in the reaction system and/or the concentration [M] of the transition metal atom of Group 8 to Group 10 derived from the compound of Group 8 to Group 10 transition metal in the reaction system is not constant, an average value obtained by weighting each concentration with time may be used as the concentration x or the concentration [M]. In addition, the reaction time t in the batch reaction usually corresponds to the total time required for the hydroformylation reaction, and when the reaction is interrupted in the middle, the total time of the actual reaction time excluding the interrupted time may be taken as the reaction time t, and the same applies to the residence time t in the continuous reaction.

[Cyclic Hemiacetal Compound (II)]

The cyclic hemiacetal compound (II) obtained by the production method of the present invention is represented by the following general formula (II).

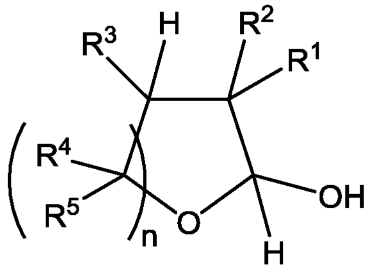

(II)

In the general formula (II), $R^1$ to $R^5$ each independently represent any one selected from the group consisting of a hydrogen atom, an optionally substituted alkyl group having 1 to 6 carbon atoms, and an optionally substituted aryl group having 6 to 12 carbon atoms. n represents 1 or 2. When n is 2, a plurality of $R^4$ and a plurality of $R^5$ may be the same as or different from each other.

Examples of the alkyl group having 1 to 6 carbon atoms include a methyl group, an ethyl group, a propyl group, an isopropyl group, a butyl group, an isobutyl group, an s-butyl group, a t-butyl group, a pentyl group, a hexyl group, a cyclopentyl group, and a cyclohexyl group. Among these, from the viewpoint of reaction activity, a methyl group and an ethyl group are preferable, and a methyl group is more preferable.

Examples of the aryl group having 6 to 12 carbon atoms include a phenyl group, a tolyl group, a 1-naphthyl group, and a 2-naphthyl group. Among these, a phenyl group is preferable.

Examples of the substituents for the optionally substituted alkyl group having 1 to 6 carbon atoms and the optionally substituted aryl group having 6 to 12 carbon atoms include a hydroxy group; an alkoxy group or an ether group such as a methoxy group, an ethoxy group, a propoxy group, a butoxy group, an isobutoxy group, a s-butoxy group, a t-butoxy group, a cyclopentyloxy group, a cyclohexyloxy group, a 2-methoxyethoxy group, a 2-ethoxyethoxy group, a furyl group, a tetrahydrofuryl group, a tetrahydropyranyl group, a phenoxy group, a benzyloxy group, a biphenyloxy group, and a naphthyloxy group; a ketone group such as an acetyl group and a benzoyl group; an aldehyde group such as a formyl group; a carboxylic acid group and a metal salt thereof; an acyloxy group such as an acetoxy group and a benzoyloxy group; a carbonate group such as a methoxycarbonyloxy group and a phenoxycarbonyloxy group; a cyano group; a sulfide group such as a methylsulfanyl group and a phenylsulfanyl group; a sulfoxide group such as a methylsulfinyl group and a phenylsulfinyl group; a sulfonyl group such as a methylsulfonyl group and a phenylsulfonyl group;

a sulfonic acid group and a metal salt thereof; a silyl group such as a trimethylsilyl group and a triphenylsilyl group; a phosphino group such as a dimethylphosphino group, a dibutylphosphino group, and a diphenylphosphino group; a phosphineoxide group such as an oxodimethylphosphino group, an oxodibutylphosphino group, and a oxodiphenylphosphino group; a phosphonate group and a metal salt thereof; and a halogen group such as a chloro group and a bromo group.

Specific examples of the cyclic hemiacetal compound (II) include 2 -hydroxytetrahydrofuran, 2-hydroxy-4-methyltetrahydrofuran, 2 -hydroxy-3-methyltetrahydrofuran, 2-hydroxy-3,3-dimethyltetrahydrofuran, 2-hydroxy-3-ethyltetrahydrofuran, 2-hydroxy-4-ethyltetrahydrofuran, 2-hydroxy-3-phenyltetrahydrofuran, 2-hydroxy-4-phenyltetrahydrofuran, 2-hydroxytetrahydropyran, 2-hydroxy-3-methyltetrahydropyran, 2-hydroxy-4-methyltetrahydropyran, and 2-hydroxy-5-methyltetrahydropyran.

[Unsaturated Alcohol (I)]

The unsaturated alcohol (I) used in the present invention is represented by the following general formula (I).

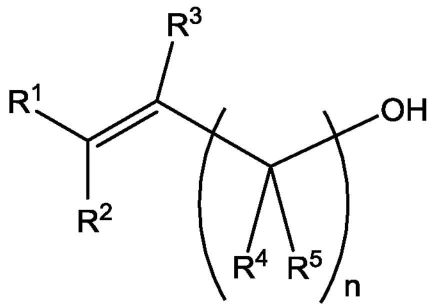

(I)

In the general formula (I), $R^1$ to $R^5$ each independently represent any one selected from the group consisting of a hydrogen atom, an optionally substituted alkyl group having 1 to 6 carbon atoms, and an optionally substituted aryl group having 6 to 12 carbon atoms. n represents 1 or 2. When n is 2, a plurality of $R^4$ and a plurality of $R^5$ may be the same as or different from each other.

Examples of the alkyl group having 1 to 6 carbon atoms include the same alkyl groups having 1 to 6 carbon atoms as those exemplified as the alkyl groups having 1 to 6 carbon atoms represented by $R^1$ to $R^5$ in the general formula (II). Among these, from the viewpoint of reaction activity, a methyl group and an ethyl group are preferable, and a methyl group is more preferable.

Examples of the aryl group having 6 to 12 carbon atoms include the same aryl groups exemplified as the aryl groups having 6 to 12 carbon atoms represented by $R^1$ to $R^5$ in the general formula (II). Among these, a phenyl group is preferable.

Examples of the substituents for the optionally substituted alkyl group having 1 to 6 carbon atoms and the optionally substituted aryl group having 6 to 12 carbon atoms include the same substituents as those exemplified as the substituents for the optionally substituted alkyl group having 1 to 6 carbon atoms and the optionally substituted aryl group having 6 to 12 carbon atoms represented by $R^1$ to $R^5$ in the general formula (II).

In the general formula (I), $R^1$ and $R^2$ are preferably a hydrogen atom from the viewpoint of reaction activity. In the general formula (I), $R^3$ is preferably an optionally substituted alkyl group having 1 to 6 carbon atoms, more preferably an unsubstituted alkyl group having 1 to 6 carbon atoms, and still more preferably a methyl group. In the general formula (I), $R^4$ and $R^5$ are preferably a hydrogen atom.

Specific examples of the unsaturated alcohol (I) include allyl alcohol, methallyl alcohol, 2-buten-1-ol, 3-methyl-2-buten-1-ol, 2-penten-1-ol, 2-ethyl-2-propen-1-ol, 3-phenyl-2-propen-1-ol, 2-phenyl-2-propen-1-ol, 3-buten-1-ol, 3-penten-1-ol, 3-methyl-3-buten-1-ol, and 2-methyl-3-buten-1-ol. Among these, from the viewpoint of reaction activity, methallyl alcohol and 3-methyl-3-buten-1-ol are preferable, and methallyl alcohol is more preferable.

[Compound of Group 8 to Group 10 Transition Metal]

The compound of Group 8 to Group 10 transition metal used in the present invention is a compound containing a transition metal of Group 8 to Group 10 of the periodic table, and may have a catalytic ability for the hydroformylation reaction by itself, or may function as a catalyst for the hydroformylation reaction by being coordinated with a tertiary phosphite ligand described later. Examples of the compound of Group 8 to Group 10 transition metal include a rhodium compound, a cobalt compound, a ruthenium compound, and an iridium compound. From the viewpoint of reaction activity, a rhodium compound and a cobalt compound are preferable, and a rhodium compound is particularly preferable.

As the rhodium compound, any rhodium compound that has a catalytic ability for a hydroformylation reaction or that changes to have a catalytic ability for a hydroformylation reaction under hydroformylation reaction conditions can be used. Specific examples of the rhodium compound include $Rh_4(CO)_{12}$, $Rh_6(CO)_{16}$, $Rh(acac)(CO)_2$, rhodium oxide, rhodium chloride, acetylacetonatorhodium, and rhodium acetate. These rhodium compounds may be commercially available, or may be synthesized by a known method using an inorganic rhodium compound such as rhodium nitrate, rhodium sulfate, rhodium trichloride, or rhodium oxide; a rhodium organic acid salt such as rhodium acetate; or various salts or carbonyl compounds of rhodium.

When the hydroformylation reaction is carried out, the concentration of the compound of Group 8 to Group 10 transition metal is preferably set to a low concentration range of 0.01 to 0.30 mM, and more preferably set to a range of 0.02 to 0.15 mM in terms of Group 8 to Group 10 transition metal atoms contained in the transition metal compound.

[Tertiary Phosphite Ligand]

The tertiary phosphite ligand used in the present invention is not particularly limited, but from the viewpoint of reaction activity, a triaryl phosphite is preferable, and a tertiary phosphite ligand represented by the following general formula (III) is more preferable.

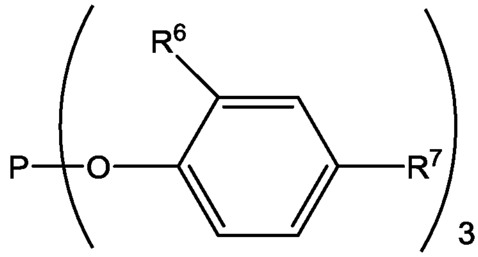

(III)

In the general formula (III), $R^6$ and $R^7$ each independently represent any one selected from the group consisting of a hydrogen atom and an optionally substituted alkyl group having 1 to 6 carbon atoms. The plurality of $R^6$ and $R^7$ may be the same as or different from each other, and are preferably the same as each other.

Examples of the alkyl group having 1 to 6 carbon atoms include the same alkyl groups having 1 to 6 carbon atoms as those exemplified as the alkyl groups having 1 to 6 carbon atoms represented by $R^1$ to $R^5$ in the general formula (II). Examples of the substituents for the optionally substituted alkyl group having 1 to 6 carbon atoms include the same substituents as those exemplified as the substituents for the optionally substituted alkyl group having 1 to 6 carbon atoms and the optionally substituted aryl group having 6 to 12 carbon atoms represented by $R^1$ to $R^5$ in the general formula (II). From the viewpoint of reaction activity, $R^6$ and $R^7$ are each preferably an optionally substituted alkyl group having 1 to 6 carbon atoms, more preferably an unsubstituted alkyl group having 1 to 6 carbon atoms, and still more preferably a methyl group, a t-butyl group, and an ethyl group.

Specific examples of the tertiary phosphite ligand include tris(2-methylphenyl)phosphite, tris(2,6-dimethylphenyl)phosphite, tris(2 -isopropylphenyl)phosphite, tris(2 -phenylphenyl)phosphite, tris(2-t-butylphenyl)phosphite, tris(2-t-butyl-5-methylphenyl)phosphite, tris(2,4-di-t-butylphenyl)phosphite, tris(2-t-butyl-4-methylphenyl)phosphite, bis(2-methylphenyl)(2-t-butylphenyl)phosphite, and bis(2-t-butylphenyl)(2-methylphenyl)phosphite. Among these, tris(2-t-butyl-4-methylphenyl)phosphite, tris(2 -t-butylphenyl)phosphite, tris(2-t-butyl-5-methylphenyl)phosphite, and tris(2,4-di-t-butylphenyl)phosphite are preferable for industrial implementation of the present invention.

The tertiary phosphite ligand may be used in only one kind, or two or more kinds thereof may be used in combination.

The tertiary phosphite ligand is preferably used in a range of 10 to 200 mol times in terms of phosphorus atom with respect to 1 atom of the transition metal of Group 8 to Group 10 contained in the compound of Group 8 to Group 10 transition metal. When the amount of the tertiary phosphite ligand used is equal to or greater than the lower limit, the selectivity in the hydroformylation reaction can be increased, and thermal degradation of the catalyst can be suppressed when the product is separated by evaporation from the reaction mixture, and a decrease in catalyst activity can be suppressed. On the other hand, when the amount of the tertiary phosphite ligand used is equal to or less than the upper limit, a decrease in the rate of the hydroformylation reaction can be suppressed.

[Base]

In the present invention, by carrying out the hydroformylation reaction in the presence of a base, the formation of a high-boiling component can be suppressed and the yield of the target cyclic hemiacetal compound (II) can be

increased. Examples of the base include inorganic bases such as sodium hydroxide, potassium hydroxide, sodium carbonate, potassium carbonate, sodium hydrogen carbonate, and potassium hydrogen carbonate; and organic bases such as a tertiary amine, a pyridine compound, a quinoline compound, and an imidazole compound. Among these, an organic base is preferable, and a tertiary amine is more preferable.

Examples of the tertiary amine include monoamines such as triethylamine, tripropylamine, trioctylamine, dimethylbenzylamine, dimethylcyclohexylamine, methyldicyclohexylamine, dimethyloctylamine, and dimethyldodecylamine; diamines such as tetramethylethylenediamine, tetramethylpropanediamine, tetramethylhexamethylenediamine, triethylenediamine, bis(dimethylaminoethyl)ether, N,N'-dimethylpiperazine, and tetramethyltoluenediamine; and triamines such as pentamethyldiethylenetriamine. The tertiary amine may have a substituent such as an alkyl group, an aryl group, a cycloalkyl group, or a hydroxyalkyl group, and may be, for example, an amino alcohol such as methyldiethanolamine, triethanolamine, methyldiisopropanolamine, or triisopropanolamine. The tertiary amine is preferably a monoamine or an amino alcohol, and more preferably trioctylamine or triethanolamine.

Examples of the pyridine compound include pyridine, picoline, lutidine, phenylpyridine, and nicotinic acid. Examples of the quinoline compound include quinoline, isoquinoline, methylquinoline, and phenylquinoline. Examples of the imidazole compound include imidazole, N-methylimidazole, 1,2-dimethylimidazole, 1-isobutyl-2-methylimidazole, 1-benzyl-2-methylimidazole, and benzimidazole.

The base may be added to the reaction solution before the hydroformylation reaction of the unsaturated alcohol (I), and the specific addition method is not particularly limited. The base may be used in such a manner that the concentration of the base in the reaction system is 500 mM (mmol/liter) or less, preferably 200 mM or less, more preferably 150 mM or less, and may be 80 mM or less, or even 50 mM or less, and preferably 1 mM or more, more preferably 5 mM or more, and may be 10 mM or more, 20 mM or more, or even 30 mM or more, within a range in which the A value and the B value satisfy the requirements of the present invention, in consideration of the production cost and the like in addition to the reaction result. When the concentration of the base in the reaction system is the above-described upper limit or less, a decrease in the reaction rate can be more effectively suppressed, and when the concentration of the base is the above-described lower limit or more, the formation of a high-boiling component can be more effectively suppressed.

[Hydroformylation Reaction]

The reaction temperature of the hydroformylation reaction is preferably in the range of 60 to 150°C, more preferably in the range of 70 to 120°C, and still more preferably in the range of 80 to 110°C. When the reaction temperature is 60°C or higher, the reaction rate is increased, and when the reaction temperature is 150°C or lower, the stability of the catalyst is easily maintained. The reaction time (or residence time in the continuous reaction) is preferably 2 to 12 hours, and more preferably 3 to 6 hours.

Carbon monoxide and hydrogen can be used, for example, as a mixed gas thereof. In the mixed gas, the molar ratio of hydrogen/carbon monoxide can be selected from the range of 1/5 to 5/1 as the incoming gas composition. In addition, the reaction system may contain a small amount of a gas inert to the hydroformylation reaction, such as methane, ethane, propane, nitrogen, helium, or argon.

The reaction pressure depends on the reaction temperature, but is preferably in the range of 5 to 20 MPaG (gauge pressure), more preferably in the range of 7 to 15 MPaG, and still more preferably in the range of 8 to 10 MPaG. When the reaction pressure is 5 MPaG or more, the selectivity of the cyclic hemiacetal compound (II) in the hydroformylation reaction can be improved. On the other hand, from the viewpoint of the apparatus used for the reaction and the operability, it is industrially advantageous to carry out the reaction while maintaining the reaction pressure at 20 MPaG or less. The reaction can be carried out batchwise or continuously in a stirred reaction tank or bubble column reaction tank.

The hydroformylation reaction can be carried out in the absence of a solvent or in the presence of an inert solvent. Examples of the solvent include alcohols such as ethanol, butanol, and ethylene glycol; saturated aliphatic hydrocarbons such as hexane, heptane, and octane; aromatic hydrocarbons such as benzene, toluene, xylene, cumene, pseudocumene, and ethylbenzene; and ethers such as tetrahydrofuran.

The cyclic hemiacetal compound (II) produced by the hydroformylation reaction can be separated from the catalyst by, for example, evaporating the reaction mixture under reduced pressure. The fraction containing the cyclic hemiacetal compound (II) obtained by the evaporation is purified by a known means such as distillation to isolate the cyclic hemiacetal compound (II).

It is also possible to recycle all or part of the compound of Group 8 to Group 10 transition metal (which may be coordinated with a tertiary phosphite ligand) contained in the evaporation residue to the hydroformylation reaction again, and reuse the same. In particular, since the rhodium compound is extremely expensive, recycling and reuse of rhodium compounds is industrially advantageous.

In addition to the target cyclic hemiacetal compound (II), unreacted starting materials, solvents, low-boiling or high-boiling by-products, and the like may be present in the reaction mixture obtained after the hydroformylation reaction.

Here, the low-boiling by-product means a compound having a boiling point lower than that of the target cyclic hemiacetal compound (II), and examples thereof include a saturated alcohol produced by hydrogenation of the unsaturated alcohol (I) as a raw material and an aldehyde compound produced by isomerization. The high-boiling by-product means a compound having a boiling point higher than that of the target cyclic hemiacetal compound (II), and examples thereof include an acetal compound produced by the reaction of hemiacetal with alcohol.

In general, the high-boiling by-products often have high viscosity, and in the case where all or part of the compound of Group 8 to Group 10 transition metal (which may be coordinated with a tertiary phosphite ligand) contained in the evaporation residue is recycled to the hydroformylation reaction and reused as described above, the content of the high-boiling by-products is desirably as low as possible from the viewpoint of the recovery rate of the target cyclic hemiacetal compound.

According to the method for producing the cyclic hemiacetal compound (II) of the present invention, when the A value and the B value defined by the above equation (1) and equation (2) are within the above ranges, the formation of a specific high-boiling component contained in the reaction mixture can be suppressed, and thus the catalyst can be easily reused. Accordingly, the target cyclic hemiacetal compound (II) can be produced more simply and in a good yield.

Examples

Hereinafter, the present invention will be specifically described with reference to Examples, but the present invention is not limited to these examples. In the Examples, gas chromatography analysis and high-performance liquid chromatography analysis were carried out under the following conditions, and the yield of the cyclic hemiacetal compound (II) and the amount of a high-boiling by-product produced were determined by an internal standard method using a calibration curve method.

[Conditions for gas chromatography analysis]

Analytical instrument: GC-2014 (manufactured by Shimadzu Corporation)
Detector: FID (hydrogen flame ionization type detector)
Column: DB-WAX (length: 30 m, film thickness: 0.25 pm, inner diameter: 0.25 mm) (manufactured by Agilent Technologies Co., Ltd.).
Analysis conditions: Injection temperature: 250°C, Detection temperature: 250°C
Temperature rise conditions: 50°C (held for 2 minutes) → (temperature rise at 8°C/min) → 160°C (held for 5 minutes) → (temperature rise at 15°C/min) → 240°C (held for 5 minutes).

[Conditions for high-performance liquid chromatography analysis]

Detector: Differential refractive index (RI) detector
Column: CHEMCOSORB 5Si (4.6 mm × 250 mm) (manufactured by Chemco Plus Scientific Co., Ltd.)
Eluent: Tetrahydrofuran/hexane = 1/1 (vol/vol)
Flow rate: 1 mL/min

<Example 1>

In a nitrogen atmosphere, a mixed solution was prepared by dissolving 2.8 g (5.4 mmol) of tris(2-t-butyl-4-methylphenyl)phosphite as a tertiary phosphite ligand and 15.5 mg (0.060 mmol) of $Rh(acac)(CO)_2$ as a compound of Group 8 to Group 10 transition metal in 150 mL of toluene (hereinafter, this mixed solution is referred to as "catalyst liquid A").

In an electromagnetic stirring autoclave having an internal volume of 100 mL and equipped with a gas inlet and a sampling port, 3.0 mL of the catalyst liquid A and 0.89 g (6.0 mmol) of triethanolamine as a base and 49 g (0.68 mol) of methallyl alcohol as an unsaturated alcohol (I) were added in a nitrogen atmosphere (hereinafter, this is referred to as a "reaction stock solution A"). At this time, the Rh concentration in the reaction stock solution A was 0.02 mM, the concentration of the tertiary phosphite ligand was 1.8 mM, and the concentration of the base (triethanolamine) was 100 mM.

The inside of the autoclave was set to 5 MPaG with a mixed gas of carbon monoxide : hydrogen = 1:1 (molar ratio), and then the temperature in the autoclave was raised to 110°C over 30 minutes while stirring. Next, the pressure was increased such that the total pressure was 9.8 MPaG, and the reaction was allowed to proceed for 5 hours to obtain a reaction mixture. During the reaction, a mixed gas of carbon monoxide : hydrogen = 1:1 (molar ratio) was constantly supplied to keep the pressure in the reaction system constant. The A value was 1.9 and the B value was 0.77.

The obtained reaction mixture was analyzed by gas chromatography to find that the conversion rate of methallyl

alcohol was 99% and the yield of 2-hydroxy-4-methyltetrahydrofuran as the cyclic hemiacetal compound (II) was 87%.

<Reference Example>

A part of the reaction mixture obtained in Example 1 was concentrated to distill off a compound having a boiling point lower than that of the cyclic hemiacetal compound (II) (2-hydroxy-4-methyltetrahydrofuran), and the residue was purified by silica gel chromatography. As a developing solvent, a mixed solvent of ethyl acetate/hexane = 1/1 (vol/vol) was used. As a result, a specific high-boiling by-product (hereinafter referred to as "high-boiling component I") was obtained. By using this, the reaction mixture obtained in Example 1 was analyzed by liquid chromatography to find that the high-boiling component I was contained in an amount of 1.0% by mass.

<Example 2 and Comparative Examples 1 and 2>

The hydroformylation reaction of methallyl alcohol was carried out under the same conditions as in Example 1 except that the concentration of the base (triethanolamine), the reaction temperature, and the reaction time were changed as shown in Table 1. The A value, B value, and reaction results at that time are shown in Table 2.

Table 1

| | Reaction temperature [°C] | Rh concentration [mM] | Concentration of tertiary phosphite ligand [mM] | Concentration of base (triethanolamine) [mM] | Reaction time [hr] |
|---|---|---|---|---|---|
| Example 1 | 110 | 0.02 | 1.8 | 100 | 5 |
| Example 2 | 110 | 0.02 | 1.8 | 20 | 4 |
| Comparative Example 1 | 90 | 0.02 | 1.8 | 2 | 5 |
| Comparative Example 2 | 110 | 0.02 | 1.8 | 2 | 4 |

Table 2

| | A value [-] | B value [-] | Conversion of methallyl alcohol [%] | Yield of cyclic hemiacetal compound (II) [%] | High-boiling component I [% by mass] | Recovery rate*1 [%] |
|---|---|---|---|---|---|---|
| Example 1 | 1.9 | 0.77 | 99 | 87 | 1.0 | A |
| Example 2 | 3.2 | 0.75 | 98 | 85 | 2.3 | B |
| Comparative Example 1 | 2.9 | 0.40 | 32 | 28 | 0.9 | A |
| Comparative Example 2 | 4.5 | 0.82 | 98 | 85 | 3.7 | C |

*1: Recovery rate of the cyclic hemiacetal compound (II) when the residue containing the Rh catalyst after the removal of the cyclic hemiacetal compound (II) from the reaction mixture by single evaporation is reused as a catalyst, and the hydroformylation reaction is further carried out 4 times (5 times in total), followed by single evaporation.

The recovery rate was evaluated according to the following criteria.

A: 90% or more
B: 80% or more and less than 90%
C: less than 80%

In Examples 1 and 2 in which the A value and the B value satisfy the requirements of the present invention, it is found that the conversion rate of methallyl alcohol was high and the yield of the cyclic hemiacetal compound (II) was

also high. In addition, the amount of the high-boiling component I produced was small, and it is found that the recovery rate of the cyclic hemiacetal compound (II) was high when the catalyst was reused and then subjected to single evaporation. On the other hand, it is found that in Comparative Example 1 in which the B value does not satisfy the requirements of the present invention, the conversion rate of methallyl alcohol was as low as 32%, and the yield of the cyclic hemiacetal compound (II) was also as low as 28%. In addition, in Comparative Example 2 in which the A value does not satisfy the requirements of the present invention, the amount of the high-boiling component I produced was as high as 3.7% by mass, and it is found that the recovery rate of the cyclic hemiacetal compound (II) was low when the catalyst was reused and then subjected to single evaporation.

<Example 3>

In a nitrogen atmosphere, a mixed solution was prepared by dissolving 16.9 g (32 mmol) of tris(2-t-butyl-4-methylphenyl)phosphite as a tertiary phosphite ligand and 92.9 mg (0.36 mmol) of Rh(acac)$(CO)_2$ as a compound of Group 8 to Group 10 transition metal in 150 mL of toluene (hereinafter, this mixed solution is referred to as "catalyst liquid B").

In an electromagnetic stirring autoclave having an internal volume of 100 mL and equipped with a gas inlet, a sampling port, a feed pump, and a gas-liquid separation tube, 3.0 mL of the catalyst liquid B and 90 mg (0.60 mmol) of triethanolamine as a base and 49 g (0.68 mol) of methallyl alcohol as an unsaturated alcohol (I) were added in a nitrogen atmosphere (hereinafter, this is referred to as a "reaction stock solution B"). At this time, the Rh concentration in the reaction stock solution B was 0.12 mM, the concentration of the tertiary phosphite ligand was 11 mM, and the concentration of the base (triethanolamine) was 10 mM.

The inside of the autoclave was set to 5 MPaG with a mixed gas of carbon monoxide : hydrogen = 1:1 (molar ratio), and then the temperature in the autoclave was raised to 90°C over 30 minutes while stirring. Next, the pressure was increased such that the total pressure was 9 MPaG, and the reaction was allowed to proceed for 1 hour. During the reaction, a mixed gas of carbon monoxide : hydrogen = 1:1 (molar ratio) was constantly supplied to keep the pressure in the reaction system constant.

Then, the catalyst liquid B, methallyl alcohol, and triethanolamine were fed at rates of 0.75 mL/hr, 12.3 g/hr and 22.5 mg/hr, respectively, and distilled from the reactor into a gas-liquid separation tube so that the liquid in the reactor was kept at 60 mL to obtain a reaction mixture. The residence time at this time was 4 hours. Further, the A value was 2.4 and the B value was 0.88.

The obtained reaction mixture was analyzed by gas chromatography to find that the conversion rate of methallyl alcohol was 78% and the yield of 2-hydroxy-4-methyltetrahydrofuran as the cyclic hemiacetal compound (II) was 70%. The high-boiling component I was contained in an amount of 1.3% by mass.

<Comparative Example 3>

The hydroformylation reaction of methallyl alcohol was carried out under the same conditions as in Example 3 except that the concentration of the tertiary phosphite ligand (tris (2-t-butyl-4-methylphenyl)phosphite), the Rh concentration, the concentration of the base (triethanolamine), and the reaction temperature were changed as shown in Table 3. The A value, B value, and reaction results at that time are shown in Table 4.

Table 3

| | Reaction temperature [°C] | Rh concentration [mM] | Concentration of tertiary phosphite ligand [mM] | Concentration of base (triethanolamine) [mM] | Residence time [hr] |
|---|---|---|---|---|---|
| Example 3 | 90 | 0.12 | 11 | 10 | 4 |
| Comparative Example 3 | 110 | 0.02 | 1.8 | 2 | 4 |

Table 4

| | A value [-] | B value [-] | Conversion rate of methallyl alcohol [%] | Yield of cyclic hemiacetal compound (II) [%] | High-boiling component I [% by mass] | Recovery rate*2 [%] |
|---|---|---|---|---|---|---|
| Example 3 | 2.4 | 0.88 | 78 | 70 | 1.3 | A |
| Comparative Example 3 | 4.5 | 0.82 | 79 | 66 | 5.5 | C |
| *2: Recovery rate of the cyclic hemiacetal compound (II) when the residue containing the Rh catalyst after the removal of the cyclic hemiacetal compound (II) from the reaction mixture by single evaporation is reused as a catalyst, and the hydroformylation reaction is further carried out 4 times (5 times in total), followed by single evaporation. | | | | | | |

The recovery rate was evaluated according to the following criteria.

A: 90% or more

B: 80% or more and less than 90%

C: less than 80%

In Example 3 in which the A value and the B value satisfy the requirements of the present invention, it is found that the conversion rate of methallyl alcohol was high and the yield of the cyclic hemiacetal compound (II) was also high. In addition, the amount of the high-boiling component I produced was small, and it is found that the recovery rate of the cyclic hemiacetal compound (II) was high when the catalyst was reused and then subjected to single evaporation. On the other hand, in Comparative Example 3 in which the A value does not satisfy the requirements of the present invention, the amount of the high-boiling component I produced was as high as 5.5% by mass, and it is found that the recovery rate of the cyclic hemiacetal compound (II) was low when the catalyst was reused and then subjected to single evaporation.

## Claims

**1.** A method for producing a hemiacetal compound represented by the following general formula (II), the method comprising reacting an alcohol having an unsaturated bond represented by the following general formula (I), carbon monoxide, and hydrogen in the presence of a compound of Group 8 to Group 10 transition metal, a tertiary phosphite ligand, and a base, wherein an A value represented by the following equation (1) is 0.0 to 4.0, and a B value represented by the following equation (2) is 0.6 to 1.0:

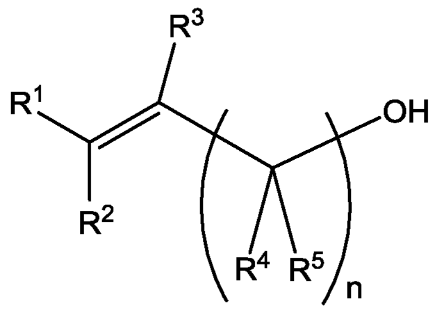

(I)

wherein $R^1$ to $R^5$ each independently represent any one selected from the group consisting of a hydrogen atom, an optionally substituted alkyl group having 1 to 6 carbon atoms, and an optionally substituted aryl group having 6 to 12 carbon atoms, and n represents 1 or 2;

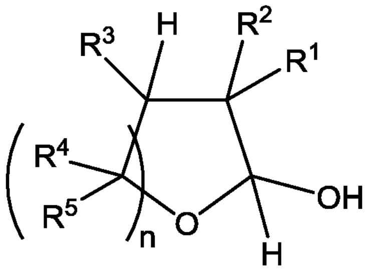

(II)

wherein $R^1$ to $R^5$ and n are as defined above;

$$A = \frac{120000 \times \exp\left(-\frac{13000}{T}\right) \times (1-0.16\ln x)}{120 \times \exp\left(-\frac{13000}{T}\right) \times (1-0.16\ln x) + 9.8 \times \exp\left(-\frac{10000}{T}\right) \times (1-0.075\ln x)} \quad (1)$$

$$B = 1 - \exp\left\{-4.9 \times 10^{12} \times \exp\left(-\frac{10000}{T}\right) \times [M] \times (1-0.075\ln x) \times t\right\} \quad (2)$$

wherein T represents a reaction temperature (K), x represents a concentration (mM) of a base in the reaction system (wherein x is 500 or less), [M] represents a concentration (mM) of a transition metal atom of Group 8 to Group 10 derived from a compound of Group 8 to Group 10 transition metal in the reaction system, and t represents a reaction time (hr) in a batch reaction or a residence time (hr) in a continuous reaction.

**2.** The method according to claim 1, wherein the compound of Group 8 to Group 10 transition metal is a rhodium compound.

**3.** The method according to claim 1 or 2, wherein $R^1$ and $R^2$ in the general formula (I) are a hydrogen atom.

**4.** The method according to any one of claims 1 to 3, wherein the alcohol having an unsaturated bond represented by the general formula (I) is methallyl alcohol or 3-methyl-3-butene-1-ol.

**5.** The method according to any one of claims 1 to 4, wherein the tertiary phosphite ligand is a triaryl phosphite.

**6.** The method according to any one of claims 1 to 4, wherein the tertiary phosphite ligand is represented by the following general formula (III):

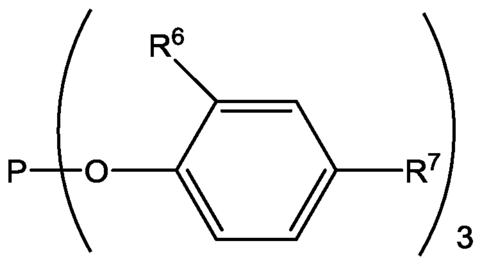

(III)

wherein $R^6$ and $R^7$ each independently represent any one selected from the group consisting of a hydrogen atom and an optionally substituted alkyl group having 1 to 6 carbon atoms.

**7.** The method according to any one of claims 1 to 6, wherein the base is a tertiary amine.

**INTERNATIONAL SEARCH REPORT**

International application No. PCT/JP2019/022241

A. CLASSIFICATION OF SUBJECT MATTER
Int.Cl. C07D307/20(2006.01)i, B01J31/28(2006.01)i, C07B61/00(2006.01)n

According to International Patent Classification (IPC) or to both national classification and IPC

B. FIELDS SEARCHED

Minimum documentation searched (classification system followed by classification symbols)
Int.Cl. C07D307/20, B01J31/28, C07B61/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 10-273465 A (KURARAY CO., LTD.) 13 October 1998, example 7, paragraphs [0015], [0018]-[0021], [0034]-[0035] (Family: none) | 1-7<br>1-7 |
| X<br>Y | JP 2001-055353 A (KURARAY CO., LTD.) 27 February 2001, claim 6, example 1, paragraphs [0028]-[0040] (Family: none) | 1-3, 5-7<br>1-7 |
| Y | JP 9-052888 A (KURARAY CO., LTD.) 25 February 1997, claims, example 1, comparative example 1, paragraphs [0013]-[0022] & US 5684167 A, claims, example 1, comparative example 1, column 3, line 7 to column 4, line 35 & EP 747373 A1 | 1-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 August 2019 (21.08.2019) | 03 September 2019 (03.09.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2019/022241

C (Continuation). DOCUMENTS CONSIDERED TO BE RELEVANT

| Category* | Citation of document, with indication, where appropriate, of the relevant passages | Relevant to claim No. |
|---|---|---|
| Y | JP 60-019781 A (KURARAY CO., LTD.) 31 January 1985, claims, examples (Family: none) | 1-7 |
| A | WO 2014/196530 A1 (KURARAY CO., LTD.) 11 December 2014, reference examples 1, 2 & US 2016/0115108 A1 & EP 3006422 A1 & CA 2914316 A & CN 105263892 A & KR 10-2016-0034249 A & HK 1218112 A & SG 11201509952P A & ES 2698832 T | 1-7 |
| A | JP 62-201881 A (KURARAY CO., LTD.) 05 September 1987, entire text & US 4808737 A & EP 223103 A1 & CA 1279326 A | 1-7 |
| A | JP 5-320087 A (TOSOH CORP.) 03 December 1993, entire text (Family: none) | 1-7 |
| A | JP 2007-320944 A (KURARAY CO., LTD.) 13 December 2007, entire text (Family: none) | 1-7 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5320087 A
- JP 9052888 A
- JP 62201881 A
- JP 11130720 A